# EUROPEAN PATENT SPECIFICATION

(11) **EP 4 635 938 B1**
(45) Date of publication and mention of the grant of the patent: **02.12.2026**
(21) Application number: 23902774.1
(22) Date of filing: 14.12.2023
(51) Int. Cl.: C07C 231/12

(54) **METHOD FOR PREPARING OSELTAMIVIR INTERMEDIATE**
VERFAHREN ZUR HERSTELLUNG EINES OSELTAMIVIR-ZWISCHENPRODUKTS
PROCÉDÉ DE PRÉPARATION D'UN INTERMÉDIAIRE D'OSELTAMIVIR

(30) Priority: 15.12.2022 CN 202211623975
(43) Date of publication of application: 22.10.2025
(73) Proprietor: Zhejiang Huahai Pharmaceutical Co., Ltd., Taizhou, Zhejiang 317024 (CN)
(72) Inventor: WANG, Wanchun, Taizhou, Zhejiang 317024 (CN); CHEN, Xinlei, Taizhou, Zhejiang 317024 (CN); LIANG, Zunjun, Taizhou, Zhejiang 317024 (CN); YAN, Fengfeng, Taizhou, Zhejiang 317024 (CN)
(74) Representative: Novagraaf International SA
(86) International application number: PCT/CN2023/138627
(87) International publication number: WO 2024/125580

(56) References cited:
- CN-A- 1 759 093
- CN-A- 109 438 276
- CN-A- 109 574 869
- CN-A- 109 574 869
- CN-A- 111 153 818
- CN-A- 111 978 195
- HARRINGTON PETER J., BROWN JACK D., FODERARO TOMMASO, HUGHES ROBERT C.: "Research and Development of a Second-Generation Process for Oseltamivir Phosphate, Prodrug for a Neuraminidase Inhibitor", ORGANIC PROCESS RESEARCH & DEVELOPMENT, AMERICAN CHEMICAL SOCIETY, US, vol. 8, no. 1, 1 January 2004 (2004-01-01), US , pages 86 - 91, XP093183393, ISSN: 1083-6160, DOI: 10.1021/op0302107
- LOU, SHAOXIA: "Research Progress in Green Chemical Methods for Deprotection of N-Boc", HUAGONG SHIKAN - CHEMICAL INDUSTRY TIMES, DONGNAN DAXUE, CN, vol. 26, no. 6, 30 June 2012 (2012-06-30), CN , pages 40 - 42, XP009555555, ISSN: 1002-154X

## Description

### FIELD OF THE INVENTION

The present application relates to the field of chemical synthesis, particularly to a preparation method of an Oseltamivir intermediate.

### BACKGROUND OF THE INVENTION

Oseltamivir phosphate is an anti-influenza drug that acts as a specific inhibitor of neuraminidase, which may inhibit mature influenza viruses from leaving host cells, thereby inhibiting the spread of influenza viruses in the body to treat influenza. It is considered to be an effective and highly specific influenza therapeutic drug at present.

The chemical name of the Oseltamivir phosphate is ethyl (3R,4R,5S)-4-acetamido-5-amino-3-(1-ethylpropoxy)-1-cyclohexene-1-carboxylate phosphate (1:1) and the structure thereof is shown as follows:

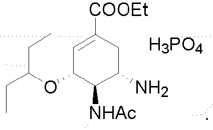

At present, there are many synthesis routes for Oseltamivir phosphate, which can be mainly divided into azide synthesis route and non-azide synthesis route. Given that the disadvantages of the azide synthesis route, such as the need to use highly toxic chemicals such as sodium azide, the high explosiveness of azides, and significant production safety pressures, the synthetic route disclosed in Chinese Patent CN100545145C is thus commonly used in the industry, which mainly comprises the opening of the oxygen ring, the synthesis of the nitrogen ring, the opening of the nitrogen ring, the acylation, the salt formation, de-tert-butylation, de-diallylation followed by formation of the phosphate. The reaction formula is as follows:

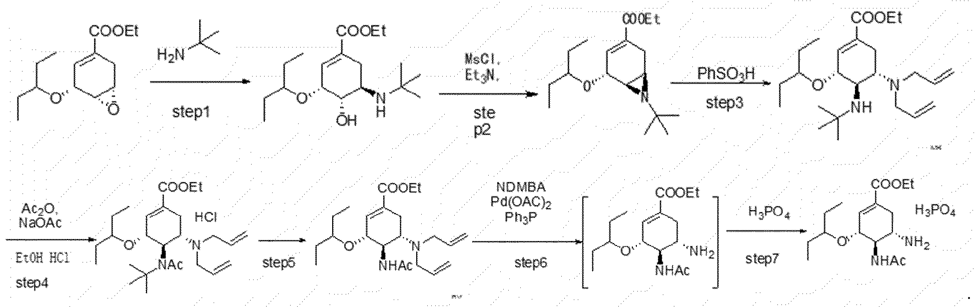

This synthesis route is a practical scheme with a superior yield, wherein trifluoroacetic acid is used for de-tert-butylation process. On the one hand, trifluoroacetic acid acts as a strong acid to catalyze the de-tert-butylation process. On the other hand, trifluoroacetic acid is used as a solvent. Therefore, a usage amount of trifluoroacetic acid is relatively large. In addition, a post-treatment after the reaction is more complicated. Firstly, trifluoroacetic acid is evaporated under vacuum, then toluene-hexane (1:1) is added, and the mixture is concentrated under vacuum. The residual oil is diluted with toluene-hexane (1:1) and then saturated sodium carbonate solution is added and the mixture is stirred for 15 minutes. The layers are separated by adding water, then the aqueous layer is extracted with toluene-hexane and diluted with toluene. The pH value is adjusted to 13 to 14 with sodium hydroxide aqueous solution. The layers are separated, the aqueous layer is extracted, and the organic layers are combined, and concentrated under vacuum to obtain a brown slurry, with purity and yield not disclosed.

Chinese Patent CN109438276A discloses a synthesis method of an intermediate compound 2 of Oseltamivir, in which trifluoroacetic acid is used for de-tert-butylation process, the solvent of the reaction is toluene, and the reaction is stirred at 45°C to 55°C. After the reaction is completed, toluene is added and the temperature is cooled to 0°C to 10°C, then cold water is added and sodium hydroxide aqueous solution is slowly added dropwise to adjust the pH value to about 12 to 13. The reaction mixture is extracted and filtered and the filtrate is concentrated in a hot water bath under reduced pressure. n-Heptane is added and the mixture is stirred for 2 hours. A crystal is then precipitated and dried.

Chinese Patent CN111253276B subjects the de-tert-butylation process using the same method, in which the usage amount of trifluoroacetic acid is 4 times that of intermediate compound 1 of Oseltamivir, toluene and water are added after the reaction is completed, the temperature is controlled at 10°C and the pH value is adjusted to 9 with 10% sodium carbonate solution. The aqueous layer is extracted with toluene for three times. Organic layers are combined and washed with water to neutrality, dried with anhydrous sodium sulfate, and concentrated and dried under reduced pressure at 60°C to obtain the product as an oil with a yield of 96.2% and a content of 97.3%.

Chinese Patent CN109438276A discloses a synthetic method of Oseltamivir, wherein acetonitrile is used as a solvent and concentrated sulfuric acid is used as a catalyst during the de-tert-butylation process. CN109574869A discloses a synthesis method of an intermediate compound 2 of Oseltamivir, in which trifluoroacetic acid is used for de-tert-butylation process, the solvent of the reaction is DCM, and the reaction is stirred at 50 °C.

According to the Newport database, the usage amount of API of Oseltamivir phosphate reached nearly 20 tons in 2021. The usage amount of API of this product is relatively large, and it is necessary to develop a cost-effective, efficient, and environmentally friendly preparation method.

### SUMMARY

Considering the shortcomings and deficiencies of the prior art, the present application provides a preparation method of an Oseltamivir intermediate, in which the original expensive reagent is replaced with lower-cost materials, uses less amount, improves molecular utilization, greatly reduces costs, and has simple post-treatment, which is very suitable for large-scale commercial production.

The present application provides a preparation method of an Oseltamivir intermediate as shown in formula 2, the method comprises the following steps:
subjecting a compound of formula 1 to a de-tert-butylation reaction under an acidic condition to obtain a compound of formula 2; 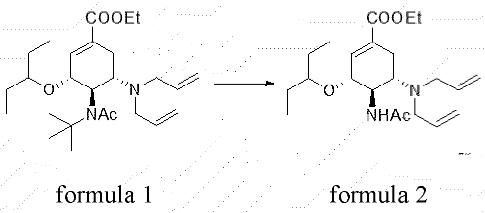
wherein the acidic condition is formed by trichloroacetic acid, chloroacetic acid, dichloroacetic acid, acetic acid, methanesulfonic acid, benzenesulfonic acid, formic acid or a mixture thereof.

In some embodiments, a reaction temperature of the de-tert-butylation reaction is 40°C to 100°C, preferably 50°C to 85°C; a reaction time of the de-tert-butylation reaction is 10 hours to 48 hours, preferably 5 hours to 14 hours.

In some embodiments, a mass ratio of the compound of formula 1 to the acid is 1:0.7 to 5, and preferably 1:0.8 to 2.0.

In some embodiments, the acidic condition is formed by formic acid, acetic acid, methanesulfonic acid or a mixture thereof. When the mixture is used, the mixture is preferably the mixture of acetic acid and methanesulfonic acid, and the mixture of formic acid and methanesulfonic acid; wherein a molar ratio of methanesulfonic acid to acetic acid or formic acid is 1:15 to 25.

In some embodiments, further comprising the following post-treatment steps to obtain the compound of formula 2: after the reaction is completed, the reaction solution is dissolved with an organic solvent to obtain a solution containing the compound of formula 2. The solution containing the compound of formula 2 is then cooled, and the pH value is adjusted to 7 to 14 (e.g., 7 to 8, 8 to 10, or between any value or any range thereof) with an inorganic alkaline aqueous solution. The reaction solution is then extracted, layered and concentrated to obtain the compound of formula 2.

In some embodiments, after the reaction is completed, the reaction solution can be concentrated under reduced pressure, then cooled to room temperature and dissolved with an organic solvent to obtain a solution containing the compound of formula 2.

In some embodiments, the organic solvent is a halogenated hydrocarbon solvent or an aromatic hydrocarbon solvent, and the volume of the organic solvent is 5 to 10 equivalents of the reaction solution.

In some embodiments, the halogenated hydrocarbon solvent is dichloromethane or dichloroethane, the aromatic hydrocarbon solvent is toluene.

In some embodiments, the temperature of the solution containing compounds of formula 2 after cooling and when adjusting the pH value is -10°C to 10°C, for example 0°C to 10°C, -10°C to 5°C, or between any value or any range thereof.

In some embodiments, the inorganic alkaline aqueous solution is selected from the group consisting of saturated sodium bicarbonate aqueous solution and sodium hydroxide aqueous solution, wherein a mass percentage concentration of the aqueous solution is 8 to 10%, preferably 10 to 15%.

As for the preparation method of Oseltamivir intermediates, the prior art has not disclosed trichloroacetic acid, formic acid, acetic acid or other used as acidic solvents for the de-tert-butylation reaction, and the usage amount of trifluoroacetic acid is large and costly. The inventor surprisingly discovered that using trichloroacetic acid, formic acid, acetic acid, methanesulfonic acid, or mixture thereof as acidic solvents may achieve the same effect while the costs are significantly reduced.

In addition, the inventor repeats the method disclosed in Chinese Patent CN109438276A and found that severe carbonization occurred after the addition of concentrated sulfuric acid, and the acetonitrile used as a solvent in the process is expensive, resulting in high overall costs.

The inventor found that when the reaction solution is added to a hydrocarbon solvent or aromatic hydrocarbon solvent, it dissolves well without the need for additional water to aid dissolution. In the post-treatment steps, the reaction solution is added dropwise to saturated sodium bicarbonate aqueous solution, at which point the reaction solution remains in a weak alkaline or neutral state, producing fewer the hydrolysis impurity and there is no need for multiple extractions and recrystallization purification. The structural formula of the hydrolysis impurity is as follows:

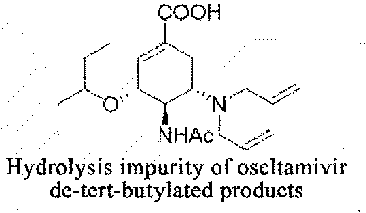

When the pH of the reaction solution is adjusted with sodium hydroxide aqueous solution, a small amount of hydrolysis product will be generated which can be removed by washing with water during post-treatment. The yield and quality of the product are essentially the same as those obtained using sodium bicarbonate.

Compared with the prior art, the present application has the following positive technical effects:
1. The preparation method provided by the present application uses weak acids such as formic acid, acetic acid and the like instead of trifluoroacetic acid, improves molecular utilization and offers clear cost advantages. This method also avoids the risk of equipment damage caused by the strong corrosive nature of strong acids, and reduces the risk of excessive heavy metals in the product.
2. The preparation method provided by the present invention uses a hydrocarbon solvent or aromatic hydrocarbon solvent in post-treatment. The solvent has a high dissolution efficiency, eliminating the need for additional water to aid dissolution.
3. The preparation method provided by the present application has a high reaction efficiency and a simple purification system, and allows for easy solvent recovery. It is green and environmentally friendly, and suitable for industrial-scale production.

### DETAILED DESCRIPTION

Specific embodiments of the present application are described in further detail below, in connection with examples. The following examples are used only to illustrate the present application, but are not intended to limit the scope of the present application.

### Example 1:

80 kg of Formic acid was charged in a four-necked flask, the mixture was heated to 75°C and then stirring was turned on. 40 kg of the compound of formula 1 was added in batches into the four-necked flask containing formic acid. The reaction was maintained at 75°C and stirred for 12 hours. HPLC was used for in-process control during the reaction. When the content of the compound of formula 1 was ≤ 0.5%, the reaction was completed. The reaction solution was maintained at 75°C and concentrated under reduced pressure to dryness. The resultant was cooled to room temperature, then dissolved in 200 L of dichloromethane under stirring. The reaction temperature was slowly cooled to 0°C to 10°C and maintained within this temperature range. The resultant was slowly added to approximately 60 kg of supersaturated sodium bicarbonate aqueous solution until the pH value was 7 to 8, and then the reaction solution was separated into layers. The aqueous layer was extracted once with 100 L of dichloromethane. The both organic layers were combined and concentrated under reduced pressure until a constant weight to obtain a yellow to white solid of the compound of formula 2, with a yield of approximately 96%, a purity of 98.8%, and 0.25% of hydrolysis impurity.

### Example 2:

90 kg of Trichloroacetic acid was charged in a four-necked flask, the mixture was heated to 75°C and then stirring was turned on. 40 kg of the compound of formula 1 was added in batches into the four-necked flask containing trichloroacetic acid. The reaction was maintained at 75°C and stirred for 14 hours. HPLC was used for in-process control during the reaction. When the content of the compound of formula 1 was ≤ 0.5%, the reaction was completed. The resultant was cooled to room temperature, then dissolved in 200 L of dichloroethane under stirring. The reaction temperature was slowly cooled to 0°C to 10°C and maintained within this temperature range. The resultant was gradually added to approximately 60 kg of the supersaturated sodium bicarbonate aqueous solution until the pH value was 7 to 8, and then the reaction solution was separated into layers. The aqueous layer was extracted once with 100 L of dichloroethane. The both organic layers were combined and concentrated under reduced pressure until a constant weight to obtain a yellow to white solid of the compound of formula 2, with a yield of approximately 92% and a purity of 97.5%.

### Example 3:

80 kg of Acetic acid was charged in a four-necked flask, the mixture was heated to 75°C and then stirring was turned on. 40 kg of the compound of formula 1 was added in batches into the four-necked flask containing acetic acid. The reaction was maintained at 85°C and stirred for 14 hours. HPLC was used for in-process control during the reaction. When the content of the compound of formula 1 was ≤ 0.5%, yielding the reaction solution. The reaction solution was maintained at 75°C and concentrated to dryness under reduced pressure. The resultant was cooled to room temperature, then dissolved in 200 L of dichloroethane under stirring. The reaction temperature was slowly cooled to 0°C to 10°C and maintained within this temperature range. The resultant was gradually added to approximately 60 kg of the supersaturated sodium bicarbonate aqueous solution until the pH value was 7 to 8, and then the reaction solution was separated into layers. The aqueous layer was extracted once with 100 L of dichloroethane. The both organic layers were combined and concentrated under reduced pressure until a constant weight to obtain a yellow to white solid of the compound of formula 2, with a yield of approximately 94.5% and a purity of 98.2%.

### Example 4:

70 kg of Formic acid was charged in a reactor kettle and then stirring was turned on. 40 kg of the compound of formula 1 was added in batches into the reactor kettle containing formic acid. The reaction was maintained at 65°C to 75°C and stirred for 7 hours to 12 hours. HPLC was used for in-process control during the reaction. When the content of the compound of formula 1 was ≤ 0.5%, the resultant was cooled to room temperature, then dissolved in 200 L of toluene under stirring. The reaction temperature was slowly cooled to -10°C to 5°C and maintained within this temperature range. The resultant was gradually added to approximately 420 kg of sodium hydroxide aqueous solution with a mass concentration of 15% until the pH value was 8 to 10, and then the reaction solution was separated into layers. The aqueous layer was extracted once with 100 L of toluene. The both organic layers were combined and washed once with 50 L of drinking water. The washed organic layer was then concentrated under reduced pressure until a constant weight to obtain a yellow to white solid of the compound of formula 2, with a yield of approximately 94.7% and a purity of 98.1%.

### Example 5:

34 kg of Formic acid was charged in a reactor kettle and then stirring was turned on. The mixture was cooled to 20°C to 30°C. Approximately 5 kg of methanesulfonic acid was slowly added dropwise. After the addition was completed, the mixture was stirred for 20 minutes to 30 minutes. 40 kg of the compound of formula 1 was added into the reactor kettle containing a mixture of formic acid and methanesulfonic acid, the reaction was maintained at the temperature of 50°C to 60°C and stirred for 5 hours to 8 hours. HPLC was used for in-process control during the reaction. When the content of the compound of formula 1 was ≤ 0.5%, the resultant was cooled to room temperature, then dissolved in 200 L of toluene under stirring. The reaction temperature was slowly cooled to -10°C to 5°C and maintained within this temperature range. The resultant was gradually added to approximately 220 kg of the sodium hydroxide aqueous solution with a mass concentration of 15% until the pH value was 8 to 10, and then the reaction solution was separated into layers. The aqueous layer was extracted once with 100 L of toluene. The both organic layers were combined and washed once with 50 L of drinking water. The washed organic layer was then concentrated under reduced pressure until a constant weight to obtain a yellow to white solid of the compound of formula 2, with a yield of approximately 95.0%, a purity of 98.5%, and 0.28% of hydrolysis impurity.

### Comparative Example 1: (referring to CN100545145C)

311 g of Trifluoroacetic acid was charged in a four-necked flask, the mixture was cooled to 0°C to 5°C and then stirring was turned on. The resultant was then added to 42.56 g of the compound of formula 1. The reaction was maintained at the temperature of 25°C and stirred for 5.5 hours. HPLC was used for in-process control during the reaction. When the content of the compound of formula 1 was ≤ 0.5%, the reaction was completed. The reaction solution was concentrated under reduced pressure to dryness at 30°C. The resultant was cooled to room temperature and diluted with 150 mL of 1:1 (v:v) toluene-hexanes under stirring. The reaction temperature was slowly cooled to 0°C to 10°C and maintained within this temperature range. 150 mL of saturated sodium carbonate aqueous solution were slowly added to the resultant followed by 150 mL of water, and the reaction solution was separated into layers. The aqueous layer was extracted with 50 mL of 1:1 (v:v) toluene-hexanes. The both organic layers were combined and then extracted twice with 2N HCl (first time 30 mL and second time 15 mL). The combined aqueous layers were diluted with 100 mL of toluene and 5.67g (142 mmol) of sodium hydroxide was dissolved in 17 mL of water until the pH=13 to 14. The layers were separated and the aqueous layer was extracted three times with 50 mL of toluene. The organic layers were combined and dried with sodium sulfate, filtered, and the filtrate was concentrated under reduced pressure to a constant weight to obtain 38.73 g of brown oily. 200 mL of hexane was added to the resultant, and then seed crystal was added. The mixture was stirred and triturated at 25°C for about 24 hours to precipitate a large amount of solids. The mass of the solids was approximately 26.76 g, with a yield of 72% and a purity of 98.0%.

**Table 1 Comparison of the results of Examples 1 to 5 and Comparative Example 1.**

| Examples | Types of acids | Mass ratio of the usage amount of the acid to the compound of formula 1 | Yield of the de-tert-butylated product | Purity of the de-tert-butylated product |
|---|---|---|---|---|
| Example 1 | Formic acid | 2 | 96% | 98.8% |
| Example 2 | Trichloroacetic acid | 2.25 | 92% | 98.2% |
| Example 3 | Acetic acid | 2 | 94.5% | 98.2% |
| Example 4 | Formic acid | 1.75 | 94.7% | 98.1% |
| Example 5 | Formic acid and methylsulfonic acid | 0.975 | 95.0% | 98.5% |
| Comparative Example 1 | Trifluoroacetic acid | 7.3 | 72% | 98% |

| | | | | |
|---|---|---|---|---|
| *Note: The price of formic acid is about 8 CNY per kilogram and the price of trifluoroacetic acid is about 72 CNY per kilogram. | | | | |

As can be seen from the results in Table 1 above, the technical solution of the present application has a cost advantage in that it uses cheaper reagents to replace expensive reagents for the reaction, but the reaction yield is not affected and there is a certain increase in yield, which makes it very suitable for large-scale industrialized production.

## Claims

1. A preparation method of an oseltamivir intermediate as shown in formula 2, wherein the method comprises the following steps: subjecting a compound of formula 1 to a de-tert-butylation reaction under an acidic condition to obtain a compound of formula 2; 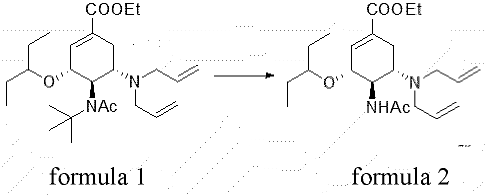 the acidic condition is formed by trichloroacetic acid, chloroacetic acid, dichloroacetic acid, acetic acid, methanesulfonic acid, benzenesulfonic acid, formic acid or a mixture thereof.

2. The preparation method according to claim 1, wherein the reaction temperature of the de-tert-butylation reaction is 40°C to 100°C, preferably 50°C to 85°C; and the reaction time of the de-tert-butylation reaction is 3 hours to 48 hours, preferably 5 hours to 14 hours.

3. The preparation method according to claim 1, wherein a mass ratio of the compound of formula 1 to the acid is 1:0.7 to 5, preferably 1:0.8 to 2.0.

4. The preparation method according to claim 1, wherein the acidic condition is formed by acetic acid, formic acid, methanesulfonic acid or a mixture thereof.

5. The preparation method according to claim 1, wherein the method further comprises the following post-treatment steps: after completing the reaction, dissolving the reaction solution with an organic solvent to obtain a solution containing the compound of formula 2; cooling the solution containing the compound of formula 2 obtained; adjusting the pH value with an inorganic alkaline aqueous solution; performing extraction and layering; and concentrating the solution to obtain the compound of formula 2.

6. The preparation method according to claim 5, wherein the solution containing the compound of formula 2 can be obtained by concentrating the reaction solution under reduced pressure after completing the reaction, followed by cooling to room temperature and then dissolving with an organic solvent.

7. The preparation method according to claim 5, wherein the organic solvent is a halogenated hydrocarbon solvent or an aromatic hydrocarbon solvent, and the volume of the organic solvent is 5 to 10 equivalents of the reaction solution.

8. The preparation method according to claim 5, wherein the pH value is 7 to 14.

9. The preparation method according to claim 5, wherein the halogenated hydrocarbon solvent is selected from the group consisting of dichloromethane and dichloroethane, the aromatic hydrocarbon solvent is toluene.

10. The preparation method according to claim 5, wherein the temperature after cooling and when adjusting the pH value is -10°C to 10°C.

11. The preparation method according to claim 5, wherein the inorganic alkaline aqueous solution is selected from the group consisting of saturated sodium bicarbonate aqueous solution and sodium hydroxide aqueous solution; wherein a mass percentage concentration of the aqueous solution is 8% to 10%, preferably 10% to 15%.

12. The preparation method according to claim 4, wherein the acidic condition is formed by acetic acid, formic acid, a mixture of acetic acid and methanesulfonic acid, or a mixture of formic acid and methanesulfonic acid; when acidic condition is formed by the mixture, a molar ratio of methanesulfonic acid to acetic acid or of methanesulfonic acid to formic acid is 1:15 to 25.

## Patentansprüche

1. Herstellungsverfahren eines Oseltamivir-Zwischenprodukts wie in Formel 2 gezeigt, wobei das Verfahren die folgenden Schritte umfasst: Unterziehen einer Verbindung der Formel 1 einer De-tert-Butylierungsreaktion unter einer sauren Bedingung, um eine Verbindung der Formel 2 zu erhalten; 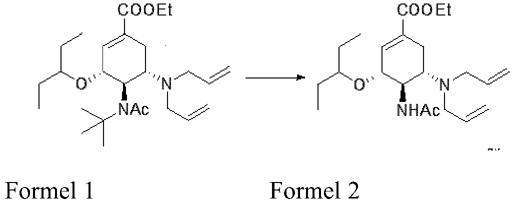 wobei die saure Bedingung durch Trichloressigsäure, Chloressigsäure, Dichloressigsäure, Essigsäure, Methansulfonsäure, Benzolsulfonsäure, Ameisensäure oder eine Mischung davon gebildet wird.

2. Herstellungsverfahren nach Anspruch 1, wobei die Reaktionstemperatur der De-tert-Butylierungsreaktion 40 °C bis 100 °C, vorzugsweise 50 °C bis 85 °C, beträgt und die Reaktionszeit der De-tert-Butylierungsreaktion 3 Stunden bis 48 Stunden, vorzugsweise 5 Stunden bis 14 Stunden, beträgt.

3. Herstellungsverfahren nach Anspruch 1, wobei ein Massenverhältnis der Verbindung der Formel 1 zu der Säure 1:0,7 bis 5, vorzugsweise 1:0,8 bis 2,0, beträgt.

4. Herstellungsverfahren nach Anspruch 1, wobei die saure Bedingung durch Essigsäure, Ameisensäure, Methansulfonsäure oder eine Mischung davon gebildet wird.

5. Herstellungsverfahren nach Anspruch 1, wobei das Verfahren ferner die folgenden Nachbehandlungsschritte umfasst: nach Abschluss der Reaktion, Auflösen der Reaktionslösung mit einem organischen Lösungsmittel, um eine Lösung zu erhalten, die die Verbindung der Formel 2 enthält; Kühlen der erhaltenen Lösung, die die Verbindung der Formel 2 enthält; Einstellen des pH-Werts mit einer anorganischen alkalischen wässrigen Lösung; Durchführen von Extraktion und Schichtung; und Konzentrieren der Lösung, um die Verbindung der Formel 2 zu erhalten.

6. Herstellungsverfahren nach Anspruch 5, wobei die Lösung, die die Verbindung der Formel 2 enthält, durch Konzentrieren der Reaktionslösung unter vermindertem Druck nach Abschluss der Reaktion, gefolgt von Kühlen auf Raumtemperatur und dann Auflösen mit einem organischen Lösungsmittel erhalten werden kann.

7. Herstellungsverfahren nach Anspruch 5, wobei das organische Lösungsmittel ein halogeniertes Kohlenwasserstofflösungsmittel oder ein aromatisches Kohlenwasserstofflösungsmittel ist und das Volumen des organischen Lösungsmittels 5 bis 10 Äquivalente der Reaktionslösung beträgt.

8. Herstellungsverfahren nach Anspruch 5, wobei der pH-Wert 7 bis 14 beträgt.

9. Herstellungsverfahren nach Anspruch 5, wobei das halogenierte Kohlenwasserstofflösungsmittel ausgewählt ist aus der Gruppe bestehend aus Dichlormethan und Dichlorethan, das aromatische Kohlenwasserstofflösungsmittel Toluol ist.

10. Herstellungsverfahren nach Anspruch 5, wobei die Temperatur nach dem Kühlen und beim Einstellen des pH-Werts -10 °C bis 10 °C beträgt.

11. Herstellungsverfahren nach Anspruch 5, wobei die anorganische alkalische wässrige Lösung ausgewählt ist aus der Gruppe bestehend aus gesättigter wässriger Natriumhydrogencarbonatlösung und wässriger Natriumhydroxidlösung; wobei eine Massenprozentkonzentration der wässrigen Lösung 8 % bis 10 %, vorzugsweise 10 % bis 15 %, beträgt.

12. Herstellungsverfahren nach Anspruch 4, wobei die saure Bedingung durch Essigsäure, Ameisensäure, eine Mischung aus Essigsäure und Methansulfonsäure oder eine Mischung aus Ameisensäure und Methansulfonsäure gebildet wird; wobei, wenn die saure Bedingung durch die Mischung gebildet wird, ein Molverhältnis von Methansulfonsäure zu Essigsäure oder von Methansulfonsäure zu Ameisensäure 1:15 bis 25 beträgt.

## Revendications

1. Procédé de préparation d'un intermédiaire d'oseltamivir tel que représenté dans la formule 2, dans lequel le procédé comprend les étapes suivantes : soumettre un composé de formule 1 à une réaction de détert-butylation en milieu acide afin d'obtenir un composé de formule 2 ; 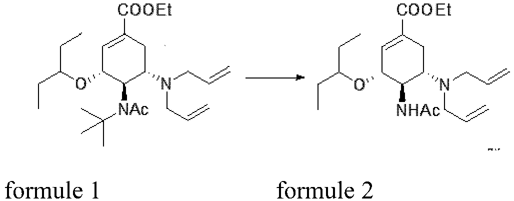 le milieu acide est formé par l'acide trichloroacétique, l'acide chloroacétique, l'acide dichloroacétique, l'acide acétique, l'acide méthanesulfonique, l'acide benzènesulfonique, l'acide formique ou un mélange de ceux-ci.

2. Procédé de préparation selon la revendication 1, dans lequel la température de réaction de la réaction de détert-butylation est de 40 °C à 100 °C, de préférence de 50 °C à 85 °C ; et la durée de réaction de la réaction de détert-butylation est de 3 heures à 48 heures, de préférence de 5 heures à 14 heures.

3. Procédé de préparation selon la revendication 1, dans lequel le rapport massique entre le composé de formule 1 et l'acide est de 1:0,7 à 5 ; de préférence de 1:0,8 à 2,0.

4. Procédé de préparation selon la revendication 1, dans lequel le milieu acide est formé par l'acide acétique, l'acide formique, l'acide méthanesulfonique ou un mélange de ceux-ci.

5. Procédé de préparation selon la revendication 1, dans lequel le procédé comprend en outre les étapes de post-traitement suivantes : après la fin de la réaction, dissoudre la solution réactionnelle avec un solvant organique pour obtenir une solution contenant le composé de formule 2 ; refroidir la solution contenant le composé de formule 2 obtenue ; ajuster le pH avec une solution aqueuse alcaline inorganique ; réaliser une extraction et une séparation des phases ; et concentrer la solution pour obtenir le composé de formule 2.

6. Procédé de préparation selon la revendication 5, dans lequel la solution contenant le composé de formule 2 peut être obtenue en concentrant la solution réactionnelle sous pression réduite après la fin de la réaction, puis en la refroidissant jusqu'à température ambiante et enfin en la dissolvant avec un solvant organique.

7. Procédé de préparation selon la revendication 5, dans lequel le solvant organique est un solvant hydrocarboné halogéné ou un solvant hydrocarboné aromatique, et le volume du solvant organique est de 5 à 10 équivalents de la solution réactionnelle.

8. Procédé de préparation selon la revendication 5, dans lequel la valeur du pH est de 7 à 14.

9. Procédé de préparation selon la revendication 5, dans lequel le solvant hydrocarboné halogéné est choisi dans le groupe constitué par dichlorométhane et dichloroéthane, le solvant d'hydrocarbure aromatique étant le toluène.

10. Procédé de préparation selon la revendication 5, dans lequel la température, après refroidissement et lors de l'ajustement de la valeur du pH, est de -10 °C à 10 °C.

11. Procédé de préparation selon la revendication 5, dans lequel la solution aqueuse alcaline inorganique est choisie dans le groupe constitué par solution aqueuse saturée de bicarbonate de sodium et solution aqueuse d'hydroxyde de sodium ; dans lequel la concentration en pourcentage massique de la solution aqueuse est de 8 % à 10 %, de préférence de 10 % à 15 %.

12. Procédé de préparation selon la revendication 4, dans lequel le milieu acide est formé par l'acide acétique, l'acide formique, un mélange d'acide acétique et d'acide méthanesulfonique, ou un mélange d'acide formique et d'acide méthanesulfonique ; lorsque le milieu acide est formé par le mélange, un rapport molaire entre l'acide méthanesulfonique et l'acide acétique ou entre l'acide méthanesulfonique et l'acide formique est de 1:15 à 25.
